# EUROPEAN PATENT APPLICATION

(11) **EP 3 868 305 A1**
(43) Date of publication of application: **25.08.2021**
(21) Application number: 20386011.9
(22) Date of filing: 19.02.2020
(51) Int. Cl.: A61B 17/00, A61B 17/29

(54) **END-EFFECTOR FOR ENDOSCOPIC SURGICAL INSTRUMENT**

(71) Applicant: UCL Business Ltd, London W1T 4TP (GB)
(72) Inventor: Stoyanov, Danail, London, N1 0JJ (GB); Marcus, Hani J., London, E14 9BT (GB); Dimitrakakis, Emmanouil, London, E2 0LR (GB)
(74) Representative: J A Kemp LLP

(57) **Abstract**

An end-effector (10) for an endoscopic surgical instrument, the end-effector comprising:
a tool (11) configured to interact with tissue;
a main body (18) comprising a bearing stud (14), the tool being connected to the bearing stud;
a base (16) comprising a surface (15) facing the bearing stud, the bearing stud and the surface forming a ball joint (12); and
a plurality of tendons connected to the main body so as to control movement of the tool in two degrees of freedom.

## Description

### Background Of The Invention

This invention relates generally to endoscopic surgery, for example endonasal neurosurgery. More particularly the present invention relates to an end-effector for an endoscopic surgical instrument, an endoscopic surgical instrument comprising the end-effector and a method of manufacturing an end-effector for an endoscopic surgical instrument.

Since the early 1980s when it was first introduced, Minimally Invasive Surgery (MIS) has had a great deal of success. Compared to traditional surgery, it requires smaller incisions, which equates to less trauma and thus reduced pain and hospital time, making MIS the standard and established procedure in a number of operations, such as laparoscopic surgery. Albeit their numerous advantages, MIS procedures are ergonomically difficult to perform due to the use of rigid instruments, visuomotor axes misalignment, limited sensory feedback and the need for high dexterity. Those drawbacks led to the development of robotic surgical devices that are now causing a paradigm shift in surgery.

Robotic-Assisted Minimally Invasive Surgery (RAMIS) has had a great impact since it allows for precise and accurate motions while reducing the learning curve for the surgeons. This means that more surgeons can perform RAMIS when they might otherwise resort to open surgery. With the introduction of robotics into the surgical theatre, a number of conventional specialties, such as urology, gynaecology, abdominal and cardiothoracic surgery, have integrated current robotic technologies into their procedures augmenting the capabilities of the surgeon while improving patient outcomes. Lately, an increasing amount of surgical procedures have deployed or started deploying robotic devices, with neurosurgery being at the forefront of these advancing technologies.

Due to its delicate subject matter and challenging operations, neurosurgery has been always in need for adapting new techniques and technologies. One such adaptation is surgical robotics, both in brain and spine applications. Although the majority of neurosurgical robots are stereotactic, technological advances in image guidance, endoscopy and laparoscopic instruments have led into the development of robotic tools for minimally invasive neurosurgery. However, the use of robotics in 'keyhole' neurosurgical approaches is still rather limited.

A number of studies have taken place implementing concentric tube robotic tools. In Burgner, J., Swaney, P.J., Rucker, D.C., Gilbert, H.B., Nill, S.T., Russell, P.T., Weaver, K.D., Webster, R.J.: "A bimanual teleoperated system for endonasal skull base surgery." (In: 2011 IEEE/RSJ international conference on intelligent robots and systems, pp. 2517-2523. IEEE (2011)), a prototype system for bimanual teleoperated endonasal skull base surgery is developed. However, there are still concerns about the distal-end dexterity of this manipulator and its grasping force and/or force-sensing capability.

It is desirable to provide an end-effector for an endoscopic surgical instrument capable of applying a greater force and/or that is more robust and/or that is capable of more dextrous manipulation of tissue and/or at a smaller size than known devices.

### Summary Of The Invention

The present invention attempts to address this desire by providing an end-effector for an endoscopic surgical instrument, an endoscopic surgical instrument comprising the end-effector and a method of manufacturing an end-effector for an endoscopic surgical instrument.

According to the invention there is provided an end-effector for an endoscopic surgical instrument, the end-effector comprising: a tool configured to interact with tissue; a main body comprising a bearing stud, the tool being connected to the bearing stud; a base comprising a surface facing the bearing stud, the bearing stud and the surface forming a ball joint; and a plurality of tendons connected to the main body so as to control movement of the tool in two degrees of freedom.

According to the invention there is provided an endoscopic surgical instrument comprising the end-effector.

According to the invention, there is provided a method of manufacturing an end-effector for an endoscopic surgical instrument, the method comprising: providing a tool configured to interact with tissue; connecting the tool to a main body comprising a bearing stud of a ball joint; and connecting a plurality of tendons to the main body to allow controlled movement of the tool in two degrees of freedom..

### Brief Description Of The Drawings

The present invention will now be described, by the way of non-limitative example only, with reference to the accompanying drawings in which:
Figure 1 shows an end-effector for an endoscopic surgical instrument according to the present invention;
Figure 2 shows the axes of degrees of freedom of the end-effector shown in Figure 1;
Figure 3 is an exploded view of the end-effector shown in Figure 1;
Figures 4 to 6 each show how the tendons control a respective degree of freedom of movement of the end-effector;
Figure 7 shows the surface of the base of the end-effector that faces the bearing stud;
Figure 8 shows the proximal end of the bearing stud that faces the surface shown in Figure 7;
Figure 9 shows the distal surface of the bearing stud that faces the tool of the end-effector;
Figures 10 to 16 show alternative arrangements of the channels for the tendons;
Figures 17 and 18 show variations of the bearing stud;
Figures 19 and 20 show alternative ways of the base of the end-effector connecting to the shaft;
Figure 21 shows alternative tools for the end-effector;
Figure 22 shows a rerouting mechanism for connecting the tendons to motors;
Figures 23 and 24 show different views of an alternative version of the main body of the end-effector that comprises the bearing stud; and
Figures 25 and 26 show different views of a further alternative version of the main body of the end-effector.

### Detailed Description

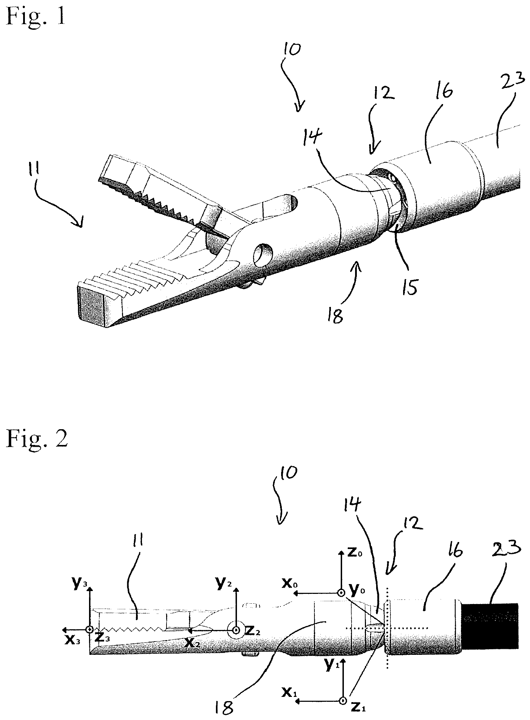

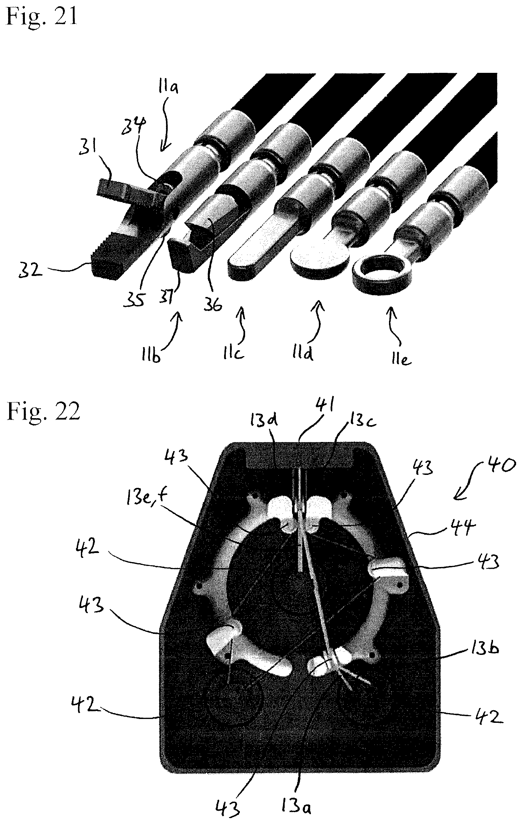

Figure 1 shows an end-effector 10. The end-effector 10 is for an endoscopic surgical instrument. As shown in Figure 1, the end-effector 10 comprises a tool 11. The tool 11 forms the tip of the end-effector 10. The tool 11 is configured to interact with tissue. For example, the tool 11 may be configured to grip, pierce, move, cut and/or dissect tissue during a surgical procedure. In the example shown in Figure 1, the tool 11 is a gripper 11a. The gripper 11a is configured to grip tissue. Other types of tools are compatible with the end-effector 10, as shown in Figure 21, for example.

As shown in Figure 1, the end-effector 10 comprises a ball joint 12. The ball joint 12 comprises a bearing stud 14 and a surface 15 facing the bearing stud 14. The ball joint 12 is configured to connect the controls of the endoscopic surgical instrument to the tool 11. As shown in Figure 1, in the ball joint 12 of the end-effector 10, it is not necessary for the bearing stud 14 to be enclosed in a casing. Instead, the bearing stud 14 may be held relative to the facing surface 15 only by the tendons 13, which are described in more detail below. The ball joint 12 allows movement of the tool 11 (e.g. changes in orientation) relative to the facing surface 15.

As shown in Figure 1, the tool 11 is connected to the bearing stud 14. For example, the tool 11 (or at least a part of it) may be fixed relative to the bearing stud 14. As mentioned above, Figure 1 shows an example in which the tool 11 is a gripper 11a. The gripper 11a may comprise an upper part 31 and a bottom part 32. The upper part 31 and the bottom part 32 can be moved relative to each other. This allows the gripper 11a to grip tissue during use. Optionally, only one of the upper part 31 and the bottom part 32 is fixed relative to the bearing stud 14. For example, Figure 1 shows an embodiment in which the bottom part 32 is connected to the bearing stud 14. The upper part 31 is connected to the bearing stud 14 only via the bottom part 32 of the gripper 11a. By providing that one of the upper part 31 and the bottom part 32 is fixed relative to the bearing stud 14, only the other (non-fixed) part is required to be controlled in order to fully activate the gripper 11a. However, this is not necessarily the case. In an alternative embodiment, both of the upper part 31 and the bottom part 32 are hinged relative to the bearing stud 14 and can be actuated independently of each other, or in unison.

By connecting the tool 11 to the bearing stud 14, movement of the bearing stud 14 relative to the facing surface 15 results in movement of the tool 11 relative to the facing surface 15. By controlling the ball joint 12, the movement of the tool 11 at the tip of the end-effector 10 can be controlled in two degrees of freedom.

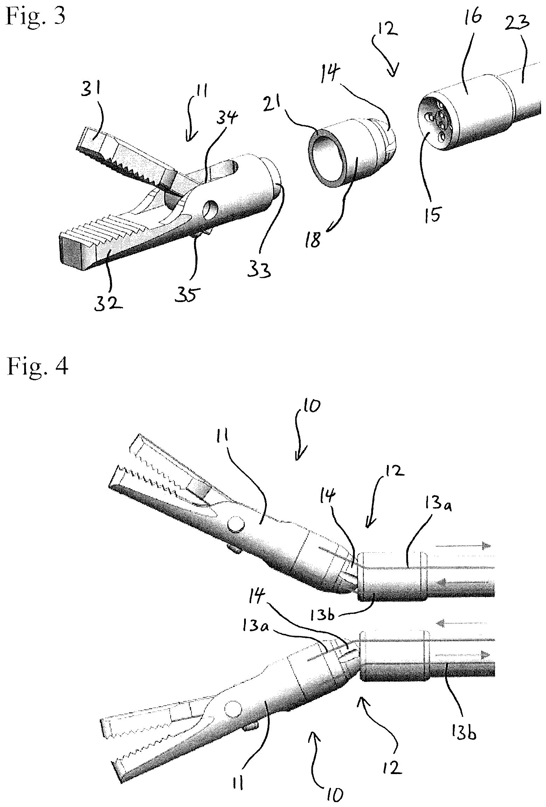

Figures 4 to 6 show how movement of the tool 11 is controlled using tendons 13. As shown in Figures 4 to 6, the end-effector 10 comprises a plurality of tendons 13. The tendons 13 are connected to the bearing stud 14. The tendons 13 are configured to control movement of the tool 11 in at least two degrees of freedom.

The invention employs a tendon-driven mechanism. By using the tendons 13 to drive the robot, the end-effector has better distal-tip dexterity and force sensing capabilities than concentric-tube robots. Furthermore, breakage and fatigue are more limited.

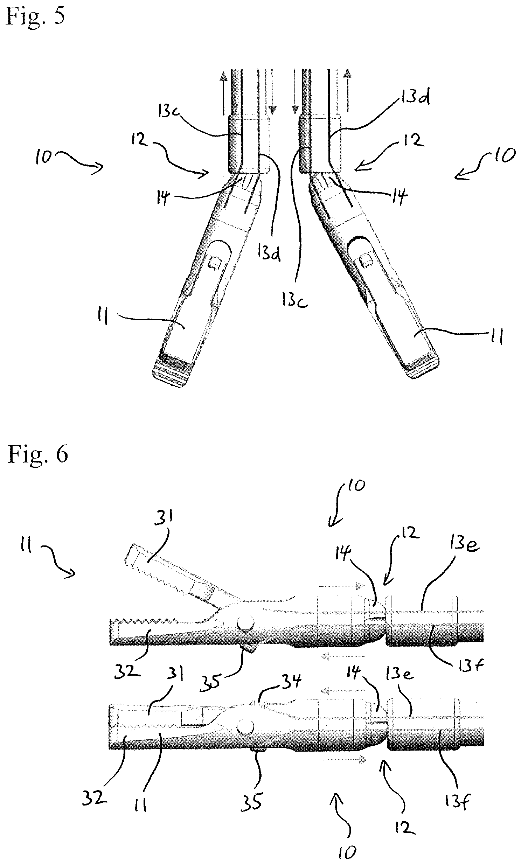

Each of Figures 4 to 6 shows how tendons 13 are used to control movement of the tool 11 in a different degree of freedom. Figures 4 and 5 show movement of the tool 11 in degrees of freedom through movement of the ball joint 12. Figure 6 shows a further degree of freedom associated with relative movement between the upper part 31 and bottom part 32 of the gripper 11a. In Figures 4 to 6 the arrows show the movement of the tendons 13.

Figure 2 shows the frames of the degrees of freedom of movement of the tool 11 on the end-effector 10. The first two degrees of freedom shown in Figures 4 and 5 have frames located on the middle point at the base of the ball joint 12. These are represented by the axes with subscripts 0 and 1. The frame of a third degree of freedom is located on the axis of rotation of the gripper 11a and is shown in Figure 2 with the subscript 2. In Figure 2, the frame with the axes x₃, y₃ and z₃ represents movement of the tip of the tool 11. Optionally, the maximum distance that the tip of the tool 11 can cover while being constrained to a fixed point, which is the origin of frames 0 and 1 as shown in Figure 2, is at least about 10mm and optionally at least about 20mm in each of the y and z axes

Optionally, each tendon 13a-d extends through the facing surface 15 to reach the bearing stud 14. As indicated in Figure 4, optionally each tendon 13a-d extends through the bearing stud 14. Alternatively, the tendons 13 may pass around the outside of the bearing stud 14. Each tendon 13a-d is connected to the main body 18 that comprises the bearing stud 14.

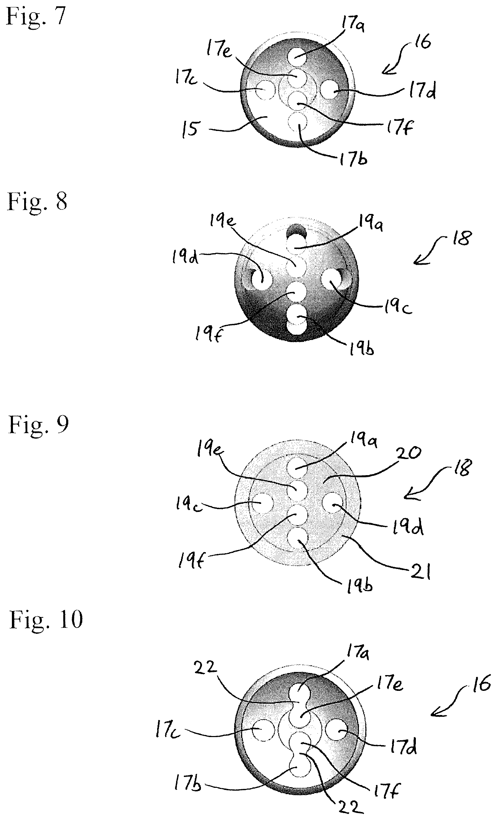

Figure 4 shows movement of the tool 11 in a degree of freedom which may be referred to as the up-down movement of the tool 11. As shown in Figure 4, this movement is controlled by tendons 13a, 13b. The tendons 13a, 13b terminate at different positions. The tendons 13a, 13b shown in Figure 4 actuate the up-down degree of freedom. Optionally, the tendons 13a, 13b terminate at a distal surface 20 (shown in Figure 9) of the main body 18 that comprises the bearing stud 14. It is not essential for the tendons 13a, 13b to terminate at the distal surface 20. For example, the tendons 13a, 13b may terminate within the channels 19 that extend through the bearing stud 14.

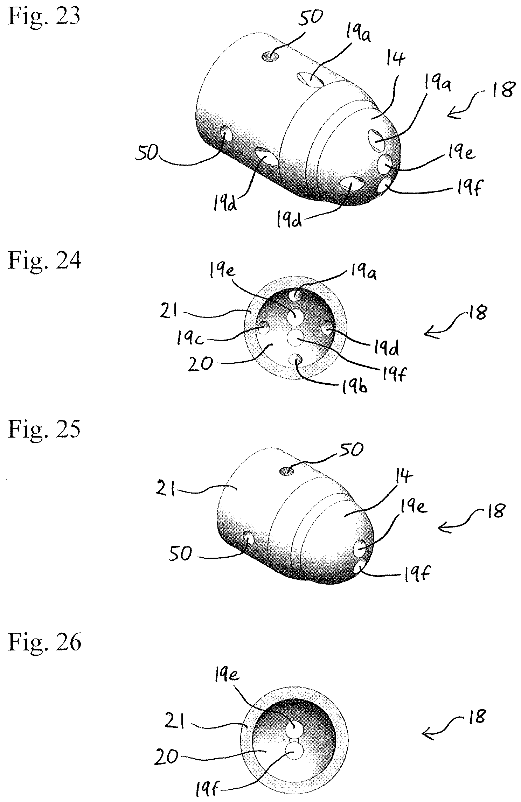

Figures 23 and 24 show different views of an alternative version of the main body 18 that comprises the bearing stud 14. Figures 25 and 26 show different views of a further alternative version of the main body 18 that comprises the bearing stud 14. As shown in the versions shown in Figures 23 to 26, optionally the tendons 13 have termination points 50 at the perimeter wall 21 of the main body 18. The tendons 13 may be fixed to the perimeter wall 21 rather than to the bearing stud 14. The tendons 13 may extend from the termination points 50 to the exterior of the main body 18. The termination points 50 may be formed as holes in the perimeter wall 21 into which the tendons 13 are fixed.

The manner in which the tendons 13a-d are fixed to their corresponding termination positions is not particularly limited. As one example, the tendons 13 may have their loose ends tied in a knot (e.g. an eight-figure knot). Alternatively, a piece of tubing may be crimped at the end of the tendon 13.

As shown in Figure 4, the tendons 13a, 13b function in an antagonistic fashion. This means that when one of the tendons 13a pulls, the other tendon 13b has released tension (lets go). This means that the tool 11 has a two-way actuation capability (e.g. up-down in the degree of freedom shown in Figure 4). The top picture shown in Figure 4 shows a situation in which one tendon 13a is pulled and the other tendon 13b is released. This causes the tool 11 to move upwards (in the orientation shown in the Figures). The bottom picture of Figure 4 shows the opposite situation in which one tendon 13a is released and the other tendon 13 is pulled. This cause the tool 11 to move downwards. The tendons 13 are maintained in tension so as to hold the bearing stud 14 to the facing surface 15.

As shown in Figure 4, the tendons in Figure 4, the tendons 13a, 13b have termination points at different positions in the up-down direction. Optionally, the tendons 13a, 13b have termination points that are at the same position in the left-right direction. Optionally, the tendons 13a, 13b have termination points that are at the same position in the axial direction.

Figure 5 shows how two other tendons 13c, 13d work in an antagonistic fashion to control movement of the tool 11. The tendons 13c, 13d control the left-right degree of freedom. In the left hand side picture of Figure 5, one tendon 13c is pulled and the other tendon 13d is released, resulting in the tool 11 moving right (when looking along the longitudinal direction of the end-effector 10 from the proximal end to the distal end where the tool 11 is positioned). In the right hand side picture of Figure 5, one tendon 13c is released and the other tendon 13d is pulled. This results in the tool 11 moving to the left.

The tendons 13c, 13d have termination positions at different points of the left-right direction. Optionally, the tendons 13c, 13d have termination points that are at the same position in the up-down direction. Optionally, the tendons 13c, 13d have termination points that are at the same position in the axial direction.

As illustrated in Figures 4 and 5, the ball joint 12 allows movement of the tool 11 in two degrees of freedom. The ball joint 12 rotates over two axes only, the pitch and yaw axes. The ball joint 12 is not required to rotate over the roll axis. Optionally, the tendons 13a-d and antagonistic motion constrain rotation of the ball joint 12 over the roll axis.

Optionally, the roll motion, as well as the translation of the tool 11, are carried out by a robot arm on which the instrument is mounted. Alternatively, the roll motion and/or the translation of the tool 11 may be carried out by the arm of a surgeon performing the procedure.

As shown in Figures 6, optionally, the end-effector 10 comprises at least one further tendon 13e, 13f. The further tendons 13e, 13f may extend through a channel 19 in the bearing stud 14. The further tendons 13e, 13f are different from the other tendons 13a-d in that they are not fixed to the bearing stud 14. The further tendons 13e, 13f are connected to the tool 11 for controlling an action of the tool 11 in a further degree of freedom.

For example, Figure 6 shows the example of a gripper 11a being used as a tool 11. The tendons 13e, 13f have termination points 34, 35 on the upper part 31 of the gripper 11a. The bottom part 32 of the gripper 11a is coupled with the main body 18 which comprises the bearing stud 14. The further tendons 13e, 13f work in an antagonistic fashion. In the top picture shown in Figure 6, one tendon 13e pulls and the other tendon 13f lets go, resulting in the gripper 11a opening (i.e. the distal portion of the upper part 31 moves away from the bottom part 32). In the lower picture of Figure 6, one tendon 13e lets go and the other tendon 13f pulls. This results in the gripper 12a closing (i.e. the distal portion of the upper part 31 moving towards the bottom part 32). Optionally, when the gripper 11a is closed, the distal portion of the upper part 31 comes into contact with the lower part 32.

Figure 3 shows an exploded view of the end-effector 10. As shown in Figure 3, optionally the end-effector 10 comprises a main body 18 that comprises a bearing stud 14. The main body 18 may be formed as a unitary piece of material. The bearing stud 14 forms a solid mass of material. Optionally, the bearing stud 14 is a unitary mass of material. This means that it is formed as a single piece, rather than a plurality of components that are later connected together. As will be explained in further detail below, the bearing stud 14 comprises a plurality of channels 19 for accommodating the tendons 13. Apart from the channels 19, the bearing stud 14 is a substantially solid piece of material without other significant holes. The bearing stud 14 is relatively dense, having a relatively large amount of material in a relatively small volume. The bearing stud 14 is robust and capable of being used to impart larger forces at the tip of the end-effector 10. In contrast, a concentric tube robot cannot apply as much force and is less robust.

By providing the end-effector 10 with the ball joint 12 for allowing movement of the tool 11, the end-effector 10 is more robust and can apply a greater force compared to known devices. Being able to apply a greater force is particularly useful for manipulating tissue with the tool 11 and/or for cutting bone. For example, a bone punch 11b is an example of a tool 11 which may be used to cut bone.

As shown in Figures 1-3, the bearing stud 14 faces the facing surface 15. Optionally, only the tendons 13 are configured to constrain the axial position of the bearing stud 14 relative to the surface 15 facing the bearing stud 14. The tendons 13 are kept in tension during use. This means that it is not necessary to encapsulate the bearing stud 14. It is not necessary to surround the bearing stud 14 in the radial direction, which helps to reduce the overall diameter of the end-effector 10. This allows the end-effector 10 to be used in smaller spaces, e.g. for endonasal neural surgery.

Optionally, the surface 15 facing the bearing stud 14 is coated with a coating configured to affect friction between the proximal end of the bearing stud 14 and the surface 15 facing the bearing stud 14. Additionally or alternatively, the proximal end of the bearing stud 14 is coated with a coating configured to affect friction between the proximal end of the bearing stud 14 and the surface 15 facing the bearing stud 14.

Optionally, the end-effector 10 comprises a frictional component between the surface 15 and the bearing stud 14 so as to affect the effective friction between the bearing stud 14 and the surface 15. Such a frictional component is not shown in the Figures. For example, a component may be inserted between the bearing stud 14 and the surface 15 so as to reduce or increase the friction that there would otherwise be if the component were not provided. The frictional component may be used to control the level of friction.

The coating or component can be chosen to control the amount of friction between the bearing stud 14 and the surface 15. Different levels of friction may be desirable for different uses of the end-effector 10 (e.g. when different levels of precision are required). It is also helpful to be able to control the level of friction in order to control the life-time of the end-effector 10.

As show in Figure 3, optionally the end-effector 10 comprises a base 16. The base 16 is formed as a separate component from the main body 18 that comprises the bearing stud 14. As shown in Figure 3, optionally the tool 11 is manufactured initially as a separate component from the main body 18. The tool 11 may then be connected to the main body 18 during the manufacturing process. For example, as shown in Figure 3 optionally the tool 11 comprises a connection element 33 that connects the tool 11 to the main body 18. Optionally, the tool 11 is fixedly connected to the main body 18. As shown in the example in Figure 3, optionally, the tool 11 comprises parts that move relative to each other. Optionally, the upper part 31 is formed as a separate component from the bottom part 32. The upper part 31 is connected to the bottom part 32 in a way that allows the distal position of the upper part 31 to move relative to the bottom part 32. As shown in Figure 3, optionally the proximal portion of the upper part 31 is connected via a hinge to the bottom part 32.

As shown in Figures 1-3, optionally the end-effector 10 is attached to a shaft 23. The shaft 23 has an internal channel through which the tendons 13 extend. Optionally, the shaft 23 extends from the end-effector 10 to the other end of the tendons 13 where they are coupled to the controls of the instrument.

Figure 7 is a close up view of the base 16 when viewed from the distal end. The surface 15 that faces the bearing stud 14 is provided at the distal end of the base 16 of the end-effector 10. Accordingly, the facing surface 15 can be seen in Figure 7. As shown in Figure 7, optionally the facing surface 15 comprises a plurality of channels 17 through which the tendons 13 extend. As shown in Figure 7, optionally a channel 17 is provided for each respective tendon 13. The correspondence between the channels 17 and the tendons 13 is indicated by the letters of the reference numerals. As can be seen in Figures 1, 3 and 7, the facing surface 15 is concave.

Figure 8 is a close up view of the main body 18 when viewed from the proximal end. The bearing stud 14 is at the proximal end of the main body 18. Accordingly, the proximal surface of the bearing stud 14 can be seen in Figure 8. As shown in Figure 8, optionally the bearing stud 14 comprises a plurality of channels 19 through which the tendons 13 extend from a proximal end of the bearing stud 14 to a distal end of the bearing stud 14. As shown in Figure 8, optionally a channel 19 is provided from each respected tendon 13. This is indicated by the letters used in the reference numerals. The channels 17, 19 reduce the possibility of the tendons 13 tangling during use.

For each rotational degree of freedom there are two tendons 13 that control the movement of each angle in an agonist-antagonist fashion. The lengths of the tendons 13 are selected by locating the 3D positions of the pass-through channels 17, 19 for every tendon 13 and calculating the distance between two consecutive channels. Optionally, the robot has six channels that tendons 13 pass through. Optionally, four of the tendons 13a-d are diametrically positioned on the same radius from the centre. Optionally, the centres of the channels 17a-d, 19a-d are positioned at least 0.5mm from the central axis of the end-effector. Optionally, the centres of the channels 17a-d, 19a-d are positioned at most 2mm from the central axis of the end-effector. Optionally, the centres of the channels 17a-d, 19a-d are positioned about 1mm from the central axis of the end-effector.

Optionally, the four tendons 13a-d terminate on the main body 18 (e.g. on the distal end of the bearing stud 14 or at the perimeter wall 21) and control the two first degrees of freedom. The remaining two tendons 13e, 13f are for controlling the degree of freedom of the gripper 11a. Optionally, the centres of the channels 17e-f, 19e-f are positioned at least 0.1mm from the central axis of the end-effector. Optionally, the centres of the channels 17e-f, 19e-f are positioned at most 0.5mm from the central axis of the end-effector. Optionally, the centres of the channels 17e-f, 19e-f are positioned about 0.35mm from the central axis of the end-effector. The tendons 13e, 13f terminate on the attachments at the upper part 31 of the gripper 11a.

During use, the difference between the lengths of the tendon 13 on the current joint-space and the desired joint-space is turned into the angle by which the respective motor should rotate in position control.

Figure 9 is a close up view of the main body 18 when viewed from the distal end. Accordingly, a distal end surface 20 of the bearing stud 14 can be seen. Additionally, a perimeter wall 21 (also indicated in Figure 3) is visible. Optionally, the perimeter wall 21 extends distally beyond the bearing stud 14. The perimeter wall 21 may be formed unitarily with the bearing stud 14. In an alternative embodiment, the main body 18 is not provided with such a perimeter wall 21. In such an embodiment, the main body 18 may be formed simply of the bearing stud 14.

As can be seen from Figures 8 and 9, the channels 19 extend from the proximal end of the bearing stud 14 (shown in Figure 8) to the distal end of the bearing stud 14 (shown in Figure 9).

As can be seen from a comparison between Figures 8 and 9, the channels 19a-d have a larger dimension at the proximal end of the bearing stud 14 compared to at the distal end of the bearing stud 14. Optionally, the channels 19a-d taper towards a distal end of the bearing stud 14. As shown in Figure 9, optionally at the distal end the channels 19a-d may be substantially circular in cross section. In contrast, as shown in Figure 8 at the proximal end of the bearing stud 14, the channels 19a-d may extend further in the radial direction of the bearing stud 14. For example, the extra size of the channels 19a-d at the proximal end of the bearing stud 14 may be achieved by providing an additional hole (e.g. an additional cylindrical hole) that joins with the main hole in forming the channels 19a-d. By providing that the channels 19a-d get smaller towards to distal end of the bearing stud 14, the tendons 13a-d can move more easily without bending at extreme angles. This improves the precision with which the movements of the tool 11 can be controlled using the tendons 13a-d.

Figures 10-16 show alternative arrangements of the tendons 13 within the channels 17, 19. Figures 10-16 are close up views of the base 16 showing the surface 15 that faces the bearing stud 14. However, the pattern of channels 17 shown in Figures 10-16 is equally applicable to the channels 19 that extends through the bearing stud 14.

As shown in Figures 10, 11, 14, 15 and 16, optionally at least one pair of channels 17 is joined to form a common channel 22 for a respective pair of tendons 13. For example, Figure 10 shows an example of which 2 channels 17a, 17e are joined together to form a common channel 22. Similarly, two further channels 17b. 17f are joined together to form another common channel 22. By joining channels 17 together to form a common channel 22, the amount of cross-sectional space required to accommodate the channels 17 can be reduced. In particular, when the tool 11 is provided with moving parts requiring tendons 13 for their control, the extra channels 17e, 17f are required. By using common channels 22,. The amount of extra space in the cross-sectional area required to accommodate the extra channels 17e, 17f can be reduced.

For tools 11 that have no moving parts, then the further tendons 13e. 13f and their respect channels 17e, 17f, 19e, 19f may not be provided. Alternatively, even when a tool 11 is used that does not have moving parts (such that the further tendons 13e, 13f are not required) the channels 17e, 17f, 19e, 19f may be provided so that the same main body 18 and base 16 can be used also for tools 11 requiring a further control tendons 13e, 13f. For example, the tool 11 could be detached from the main body 18 and replaced with another tool 11 that requires a different number of tendons 13 for control.

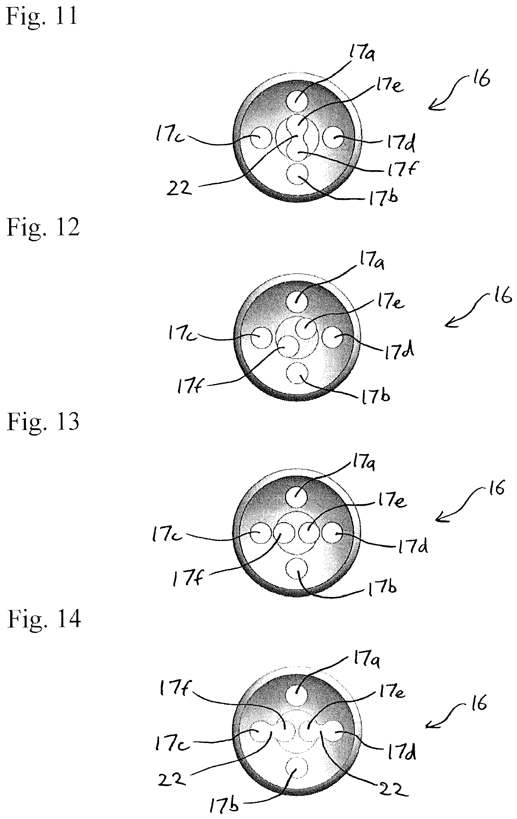

Figure 11 shows an example in which the additional channels 17e, 17f for the further tendons 13e, 13f that control an action of the tool 11 are joined to form a common channel 22. As shown Figures 10 and 11, for example, the common channel 22 may have a Figure of 8 shape.

As shown in Figures 7-11, optionally, the channels 17e, 17f, 19e, 19f for the further tendons 13e, 13f (that control an action of the tool 11) align in cross-section with a direction of movement of the further degree of movement. In particular, in the example of a gripper 11a as shown in Figures 1-3 , the further degree of freedom is in the up-down direction. This is because the further degree of freedom is movement of the upper part 31 if the gripper 11a in the up-down direction. The further channels 17e, 17f are arranged in the up-down direction. By aligning the arrangement of the channels 17e, 17f with the degree of freedom, the possibility of the further tendons 13e, 13f tangling is reduced.

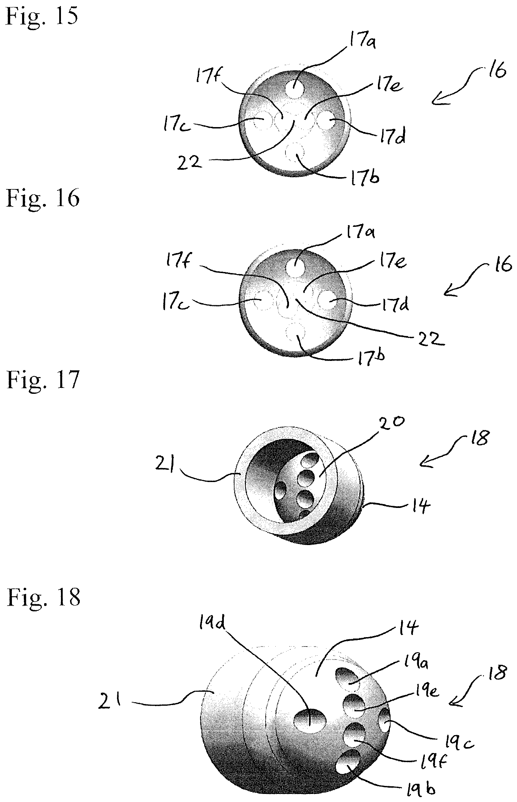

However, it is not essential for the further channels 17e, 17f to align with the further degree of freedom As shown in Figure 13,m in an alternative embodiment the further channels 17e, 17f are aligned with a direction of movement of the left-right degree of freedom. This is also shown in Figures 14 and 15. Optionally, the further channels 17e, 17f align in cross-section with a direction of movement of one of the two degrees of freedom enabled by the ball joint 12. In the example shown in Figures 1-3, one of the two degrees of freedom enabled by the ball joint 12 aligns with the further degree of freedom associated with the action of the tool 11. However, this is not the necessarily the case. For example, the action of the tool 11 could be offset from the two degrees of freedom associated with the ball joint 12.

As shown in Figures 12 and 16, optionally, the channels 17e, 17f are aligned diagonally in the cross-sectional view. This may be particularly appropriate when the further degree of freedom associated with the action of the tool 11 is aligned in the diagonal direction. Alternatively, the further channels 17e, 17f may be provided in the diagonal direction (as shown in Figures 12 and 16) so as to increase a distance between the edges of the channels 17.

As shown in Figure 14, optionally, the channels 17c, 17d associated with the left-right degree of freedom join with the further channels 17e, 17f to form joint channels 22.

Figures 23 and 24 show different views of an alternative version of the main body 18 that comprises the bearing stud 14. Figure 23 is a perspective view. Figure 24 is a view from the distal side of the main body 18. As shown in Figures 23 and 24, optionally, the channels 19a-d continue through the perimeter wall 21 of the main body. This allows the tendons 13a-d to continue around the outside of the main body 18. The tendons 13a-d may be fixed at one end to the perimeter wall 21 at the termination points 50.

Figures 25 and 26 show different views of a further alternative version of the main body 18 that comprises the bearing stud 14. Figure 25 is a perspective view. Figure 26 is a view from the distal side of the main body 18. As shown in Figures 25 and 26, optionally, the main body 18 is not provided with the channels for the tendons 13a-d. Instead, the tendons 13a-d extend around the outside of the bearing stud 14. This simplifies the design of the bearing stud 14. The tendons 13a-d may be fixed at one end to the perimeter wall 21 at the termination points 50.

Optionally, the channels 17, 19 have an internal width of at least 1 and a half times a cross-section dimension of the tendons 13. This reduces the possibility of the tendons 13 getting stuck within the channels 17, 19, thereby restricting their axial movement. Optionally, the channels 17, 19 have an internal width at least two times the cross-sectional dimension of the tendons 13.

Optionally the end-effector 10 has a diameter such that it can fit through a nostril. Optionally the end-effector 10 has a diameter such that two or three such end-effectors 10 can fit through the same nostril simultaneously. Optionally, the end-effector 10 has a maximum external diameter of at most about 7mm and optionally at most about 5mm. Optionally, the end-effector 10 has a maximum external diameter of about 3.6mm. Optionally, the end-effector 10 has a length of at most about 20mm.

Optionally, the end-effector 10 is made of medical grade steel. Optionally, the tendons 13 are made of stainless steel. However, other materials are possible. For example, the tool 11, main body 18 and base 16 may be made of plastic. Optionally, the tendons 13 are made of tungsten.

Optionally, the tendons 13 have a diameter of at most about 0.5mm and optionally at most about 0.2mm. Optionally, the channels 17, 19 have a diameter of at most about 1mm and optionally at most about 0.5mm. Optionally, the channels 17, 19 have a diameter of at least about 0.2mm and optionally at least about 0.5mm.

Optionally, the end-effector 10 is fixed on the shaft 23. The shaft 23 may be a stainless steel grade 316 seamless tube shaft. Optionally, the shaft 23 has an outer diameter of at most 7mm, and optionally at most 5mm. Optionally, the shaft 23 has an outer diameter of 3mm. Optionally, the shaft 23 comprises wire-guiding tubes through which the tendons 13 pass. Optionally, the wire-guiding tubes have a diameter of about 0.5mm

As shown in Figure 9, optionally, the bearing stud 14 comprises a flat surface 20 at its distal end. Optionally, the tendons 13 are attached at the flat surface 30. Optionally, the bearing stud 14 has a substantially hemispherical shape, with a convex surface at its proximal end (facing the facing surface 15) and a flat surface 20 at its distal end. By providing the bearing stud 14 as a substantially hemispherical object, the end-effector 10 is easier to manufacture.

However, it is not essential for the bearing stud 14 to have a hemispherical shape. Figure 17 shows an alternative version of the main body 18 in which the bearing stud 14 has a spherical shape. The distal end surface 20 of the bearing stud 14 is convex.

As mentioned above, optionally the channels 19 are wider at the proximal end of the bearing stud 14 compared to at the distal end of the bearing stud 14. However, this is not necessarily the case. Figure 18 shows an alternative version of the main body 18 in which the channels 19 have a consistent cross-section or width from the proximal end to the distal end of the bearing stud 14. In particular, Figure 18 shows that the additional holes that can be seen in Figure 8 (and can also be seen in Figures 1 to 6) are not provided.

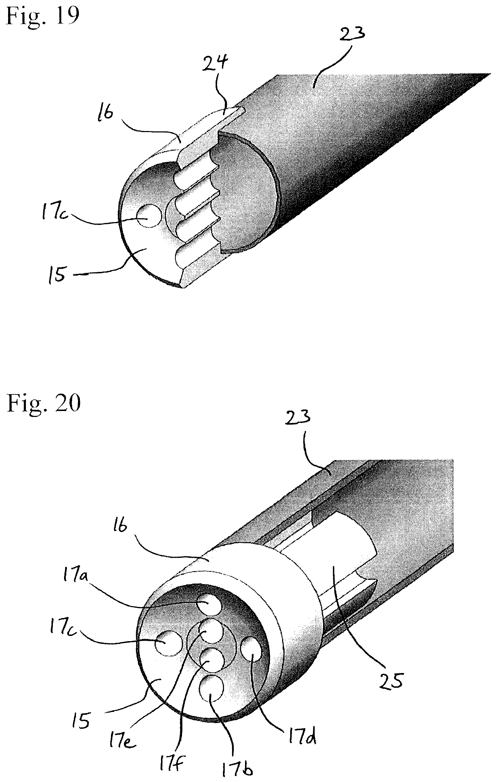

As shown in Figures 1-3, optionally the base 16 is attached to a shaft 23 through which the tendons 13 extend. Figures 19 and 20 show alternative ways of connecting the base 16 of the end-effector 10 to the shaft 23. As shown in Figure 19, optionally the base 16 comprises a flange 24 at its proximal end. The flange 24 fits around the shaft 23 so as to connect the base 16 to the shaft 23. Optionally, the connection between the end-effector 10 and the shaft 23 can be undone without breaking the end-effector 10. This allows the end-effector 10 to be replaced (e.g. if a different tool 11 is required). Alternatively, the connection between the end-effector 10 and the shaft 23 is permanent (e.g. by welding) such that the end-effector 10 cannot be replaced without damaging the end-effector 10 and/or the shaft 23.

Figure 20 shows an alternative version in which the base 16 comprises a protruding part 25 that extends within the inner channel of the shaft 23. As can be seen in Figure 20, optionally the channels 17 extend all the way through the base 16 including the protruding part 25. The overall diameter of the protruding part 25 may be so small that the channels 17a-d are exposed to the internal wall of the shaft 23.

In an alternative version, the base 16 is fixed directly onto the shaft 23 without the need for the protruding part 25. For example, optionally the base 16 is welded (e.g. laser welded) onto the shaft 23.

As mentioned above, different types of tool 11 can be used with the end-effector 10. Optionally, one tool 11 can be replaced with another without replacing the rest of the end-effector 10 (e.g. the main body 18 and the base 16). Alternatively, it may be that the whole end-effector 10 is replaced when it is desired to use a different tool 11.

The left-most tool 11 show in Figure 21 is a gripper 11a as shown in Figures 1-6. The next tool 11 is a bone punch 11b. The bone punch 11b has a moving part. In particular, the bone punch 11b requires relative axial movement between an upper part 36 and a bottom part 37. The bone punch 11b is configured to cut bone. The relative movement between the upper part 36 and the bottom part 37 of the bone punch 11b can be actuated by use of the further tendons 13e, 13f. Optionally, the bone punch 11b is spring-loaded. The middle tool 11 shown in Figure 21 is a flat-long spatula dissector 11c. The fourth tool 11 shown in Figure 21 is a round spatula dissector 11d. The right-most tool shown in Figure 21 is a ring curette 11e.

The tendons 13 extend from their termination points (at their distal end) through the shaft 23 to their proximal end. At their proximal end, the tendons 13 are attached so that they can be controlled by motors. Figure 22 shows a rerouting mechanism 40 for the tendons 13 at their proximal end.

As shown in Figure 22, optionally, the rerouting mechanism 40 comprises a housing 44. Optionally, the housing 44 is produced via additive manufacturing. Optionally, the housing 44 is made of polylactic acid (PLA) plastic. The housing 44 comprises a connection point 41 for connecting to the shaft 23. The tendons 13 extend through the shaft 23 and through the connection point 41 into the rerouting mechanism 40.

As shown in Figure 22, the rerouting mechanism 40 comprises a plurality of capstans to which the tendons 13 are connected. The capstans, in turn, are connected with stainless steel rods that are then connected to motors for actuating the tendons 13. As shown in Figure 22, optionally the rerouting mechanism 40 comprises a plurality of pulleys 43. The pulleys 43 allow the tendons to be rerouted at different axial positions of the capstans 42.

The end-effector 10 of the invention can be applied in various different types of surgery. For example, the end-effector is suitable for use in neurosurgery. However, the invention is not limited to neurosurgery. The end-effector 10 can be used in other types of surgery, such as spinal surgery.

The invention is described above mainly in the context of an end-effector 10 with six tendons 13. However, a different number of tendons 13 may be provided. For example, when the tool 11 does not require activation (e.g. when the tool 11 has no moving parts), then the end-effector 10 may comprise only four tendons for controlling the two degrees of freedom. Optionally, instead of two tendons 13 for each degree of freedom, the end-effector 10 comprises one tendon 13 per degree of freedom. For example, the tendon 13 may be looped (e.g. around a capstan), with both ends of the tendon 13 attached at different termination points for controlling movement in the degree of freedom. As another example, when a gripper 11a having both an upper part 31 and a bottom part 32 independently actuatable, then the end-effector 10 may comprise eight tendons 13 (two pairs for the up-down and left-right degrees of freedom and a pair for each part of the gripper 11a). The number of tendons 13 is not particularly limited.

Optionally, the end-effector 10 is manufactured by additive manufacturing, for example stereolithography, or direct laser metal sintering. Optionally the printing material is a medical grade steel or an autoclavable resin (of the type used in fluidics, optics and mold-making).

The Figures and previous description relate to preferred features by way of illustration only. It should be noted that alternative features of the structures and methods disclosed herein will be readily recognised as possible alternatives. The equivalent described above is by way of example only, and it will be appreciated that it may be modified in several different ways while remaining in the scope of the claims. Features of the different embodiments and arrangements may be combined with each other, except where these are mutually exclusive.

## Claims

1. An end-effector for an endoscopic surgical instrument, the end-effector comprising:
a tool configured to interact with tissue;
a main body comprising a bearing stud, the tool being connected to the bearing stud;
a base comprising a surface facing the bearing stud, the bearing stud and the surface forming a ball joint; and
a plurality of tendons connected to the main body so as to control movement of the tool in two degrees of freedom.

2. The end-effector of claim 1, wherein the bearing stud is a unitary mass of material.

3. The end-effector of claim 1 or 2, wherein the surface facing the bearing stud and/or a proximal end of the bearing stud is coated with a coating configured to affect friction between the proximal end of the bearing stud and the surface facing the bearing stud.

4. The end-effector of any preceding claim, comprising a frictional component between the surface facing the bearing stud and the bearing stud so as to affect effective friction between the bearing stud and the surface facing the bearing stud.

5. The end-effector of any preceding claim, wherein only the tendons are configured to constrain an axial position of the bearing stud relative to the surface facing the bearing stud.

6. The end-effector of any preceding claim, wherein the bearing stud comprises a plurality of channels through which the tendons extend from a proximal end of the bearing stud to a distal end of the bearing stud.

7. The end-effector of claim 6, wherein the channels taper towards the distal end of the bearing stud.

8. The end-effector of any preceding claim, comprising at least one further tendon extending through a channel in the bearing stud and connected to the tool for controlling an action of the tool in one further degree of freedom.

9. The end-effector of claim 8, wherein at least two of said further tendons are provided, having respective channels that, in cross-section, align with a direction of movement of said one further degree of freedom and/or a direction of movement of one of said two degrees of freedom.

10. The end-effector of any of claims 6 to 9, wherein at least one pair of the channels is joined to form a common channel for a respective pair of the tendons.

11. The end-effector of any of claims 6 to 10, wherein the channels have an internal width at least one and a half times a cross-sectional dimension of the tendons.

12. The end-effector of any preceding claim, wherein the main body comprises a perimeter wall extending distally relative to the bearing stud, wherein the tendons are attached to the perimeter wall.

13. The end-effector of any preceding claim, wherein the bearing stud comprises a flat or convex surface at its distal end to which the tendons are attached.

14. An endoscopic surgical instrument comprising the end-effector of any preceding claim.

15. A method of manufacturing an end-effector for an endoscopic surgical instrument, the method comprising:
providing a tool configured to interact with tissue;
connecting the tool to a main body comprising a bearing stud of a ball joint; and
connecting a plurality of tendons to the main body to allow controlled movement of the tool in two degrees of freedom.
